# EUROPEAN PATENT APPLICATION

(11) **EP 2 255 819 A1**
(43) Date of publication of application: **01.12.2010**
(21) Application number: 09161075.8
(22) Date of filing: 26.05.2009
(51) Int. Cl.: A61K 35/56

(54) **Extract of annelid and use thereof for the regeneration of the skin**

(71) Applicant: Inmobiliaria Algeciras Ltda, 2520000 Viña del Mar (CL); Melinao Canales, Victor, Temuco (CL)
(72) Inventor: Melinao Canales, Víctor Patricio, 4780000, Temuco (CL)
(74) Representative: ABG Patentes, S.L.

(57) **Abstract**

The invention relates to an extract of an annelid of the *Lumbricidae* family and, more specifically, of the species *Allolobophora caliginosa,* as well as to pharmaceutical and cosmetic compositions comprising said extract and to the uses thereof for promoting tissue regeneration and the healing of wounds as well as in cosmetic methods.

## Description

### Field of the Invention

The invention is comprised within the field of natural pharmaceutical preparations and, more specifically, within the field of preparations suitable for the treatment of dermatological diseases and, in particular, of diseases requiring the regeneration of the skin such as burns, wounds and the like. The invention also relates to a process for obtaining said pharmaceutical preparations.

### Background of the Invention

A scar can be defined as an abnormal deposition of fibrous components, mainly matrix products such as collagen and fibronectin, in the site of a wound. This deposition of fibrous tissue is more abundant than in normal skin. The result of this deposition is a granular surface or "lump", a common feature of a scar tissue. Apart from its visual difference compared to normal skin, a scar tissue also differs from normal skin in its biomechanical properties. Scar tissue, like other highly fibrous tissues, is less flexible and is usually weaker in tensile strength. This flexibility loss and weakness are what usually lead to the loss of the function of the tissue. For example, the scars of the hand, especially close to the joints, often lead to a restricted movement, since the healed skin cannot be stretched with the movement of the joint.

The production of scar tissue as a sequela of the healing of wounds or traumas is a well known process which has frequently been considered as an inevitable consequence of the healing process. In many cases, the scar tissue formed during the healing process is not a matter of great medical or social concern. However, there are many cases in which the production of scar tissue has both medical and social consequences for the individual. In those cases in which the beginning of the trauma in the patient includes areas of the body which are exposed to the public such as the face, arms or neck, scar formation can have long-lasting physiological and social implications for the patient. The physiological impact of a disfiguring facial scar due to a trauma event can be devastating or at least disturbing in some people, depending on the severity of the disfiguration and on the physiological constitution of the subject. In certain cases, a disfiguring scar can have not only a social stigma associated with it, but also an economic loss in the cases in which the personal appearances are an important aspect. Many times, it is often necessary or desirable to perform reconstructive surgery to eliminate the scar tissue for appearance reasons. This need for surgery is a costly process in economic terms as well as with regard to the pain and suffering which the patient must withstand. It would be a great advantage to have an available treatment which prevents the need for reconstructive surgery.

Additionally, there are cases in which scar tissue formation from a healing process or from a trauma has medical consequences in addition to the potential ugliness of the scar formation. In these cases, scar tissue formation can inhibit the normal physiological function of a tissue from performing its normal functions. Such example would be the case of a widely extended scar as that observed in the cases of severe burns. The patients who survive and recover from widely extended burns or who have experienced burns in critical areas such as the hands or the face often have a scar tissue which greatly reduces their capacity to recover the normal movement and functionality of the affected areas. Such cases often require repeated surgery in order to allow the patient to recover the functionality of the affected area and, in many cases, this reconstructive surgery is only partially effective, leaving the patient with a certain degree of permanent impairment. It would be a great medical advantage to have an agent which reduces scar tissue formation during the normal healing process.

Usual methods for the treatment of wounds in humans and animals involve the healing or elimination of the dead or infected tissues), disinfection using disinfectants based on iodine or on hydrogen peroxide and treatment with antibiotics (local or systemic, in the form of powder, cream or aerosol). Finally, for the purpose of preventing the hardening of the skin and scab formation, gauzes impregnated in oily wetting agents such as petroleum jelly, silicone oils or glycerol are applied. The wound is covered with sterile gauze not only to prevent the exposure of the wound to infectious agents but also to collect the exudate and the secretions of the wound.

The topical treatment of wounds is carried out today by means of the use of bacitracin, gentamicin, kanamycin, tetracycline, oxytetracycline, meclocycline, polymyxin, nitrofurazone, sulfadiazine, fusidic acid and the like. In fact, there is a great demand for new and superior agents promoting the healing of wounds.

Although there are both commercial products and agents in research stage which have shown positive effects in the healing of wounds in human beings (Meier et al., 2006, Expert Opin Emerg Drugs; 11: 23-37., Nayak et al., 2006, . BMC Complement Altern Med 6, 12), the heterogeneity of the clinical conditions accompanying the healing process of wounds in skin, as well as the particularities of the human species with regard to tissue repair and regeneration (Broughton et al., 2006, Plast Reconstr Surg; 117(7 Suppl):1e-S-32e-S; Edwards et al., 2006, Nurs Stand 2006; 21: 48-56 and Eming et al., 2007, J Invest. Dermatol.; 127:514-525), make the healing of wounds continue to represent a challenge for biomedical research, and therefore a need and a market open to new solutions.

Therefore, there is a need in the art for compositions with the capacity to promote the healing of wounds.

### Summary of the Invention

In a first aspect, the invention relates to an extract of an annelid of the *Lumbricidae* family.

In another aspect, the invention relates to a method for obtaining an extract of an annelid of the *Lumbricidae* family comprising the steps of
(i) maintaining at least one annelid of the *Lumbricidae* family at a temperature of 20 to 25°C and
(ii) collecting the liquid released by the annelid

In another aspect, the invention relates to an extract obtained by means of a method according to the second aspect of the invention.

In another aspect, the invention relates to a pharmaceutical composition comprising an extract of annelid of the invention and a pharmaceutically acceptable excipient.

In another aspect, the invention relates to an extract according to the invention or a pharmaceutical composition according to the invention for its use in medicine.

In another aspect, the invention relates to an extract according to the invention or a pharmaceutical composition according to the invention to promote the healing of a wound.

In another aspect, the invention relates to a cosmetic method for regenerating the skin comprising administering to an individual an extract according to the invention.

Finally, the invention relates to a material for treating a wound wherein said material comprises an extract according to the invention.

### Brief Description of the Drawings

Figure 1 shows the weight of the animals subjected to incision surgery.
Figure 2 shows the final size of the wound of animals subjected to incision surgery (*p<0.05, Mann-Whitney test).
Figure 3 shows photographs of animals subjected to incision surgery. Images obtained on days 0 (A), 1 (C) and 10 (E) in a rat of the control group and in a rat treated with extract (B, D and F, respectively).
Figure 4 shows microphotographs of 5 µm thick rat skin sections stained with hematoxylin-eosin. A and B: intact skin sections of control rats and rats subjected to treatment, respectively. C and D: skin wound sections of control rats. E and F: skin wound sections of rats subjected to a treatment with extract.
Figure 5 shows the immunohistochemistry for KI-67 in epidermis of a control rat (A) and a rat treated with extract (B).
Figure 6 shows the count of cells labeled positively with the antigen KI-67 in the epidermis of the wound area and the intact area of the skin.
Figure 7 shows microphotographs of 5 µm thick rat skin sections stained with picrosirius red. A and B: intact skin sections of control rats and rats subjected to treatment, respectively. C and E: skin wound sections of control rats (at low and high magnification, respectively). D and F: skin wound sections of rats subjected to treatment with extract (at low and high magnification, respectively).
Figure 8 shows the skin collagen content of animals subjected to incision surgery, measured by morphometry with picrosirius red.
Figure 9 shows serum levels of C-reactive protein.
Figure 10 shows the weight of the animals subjected to excision surgery.
Figure 11 shows the wound area of the animals subjected to excision surgery. The difference between the averages of both groups was statistically significant in each measure from day 3 onwards.
Figure 12 shows the ratio between the wound areas of the groups which received extract and the controls.
Figure 13 shows photographs of an animal of the control group during day 0 (A), day 3 (B), day 9 (C), day 15 (D), day 21 (E) and day 27 (F) .
Figure 14 shows photographs of an animal of the extract group during day 0 (A), day 3 (B), day 9 (C), day 15 (D), day 21 (E) and day 27 (F) .
Figure 15 shows the wound closure day in both groups of experimental animals. *p<0.05, Mann-Whitney test.
Figure 16 shows the microphotograph of 5 µm thick histological sections of skin wounds by excision stained with hematoxylin-eosin. A and B: skin sections showing the presence of chronic inflammation foci under the dermis and the regeneration of the muscle layer in a control rat and a rat subjected to treatment, respectively. C and D: skin sections showing the degree of indemnity of the skin annexes in a rat of the control group and a rat treated with extract, respectively.
Figure 17 shows the skin collagen content of animals subjected to excision surgery, measured by morphometry with picrosirius red.

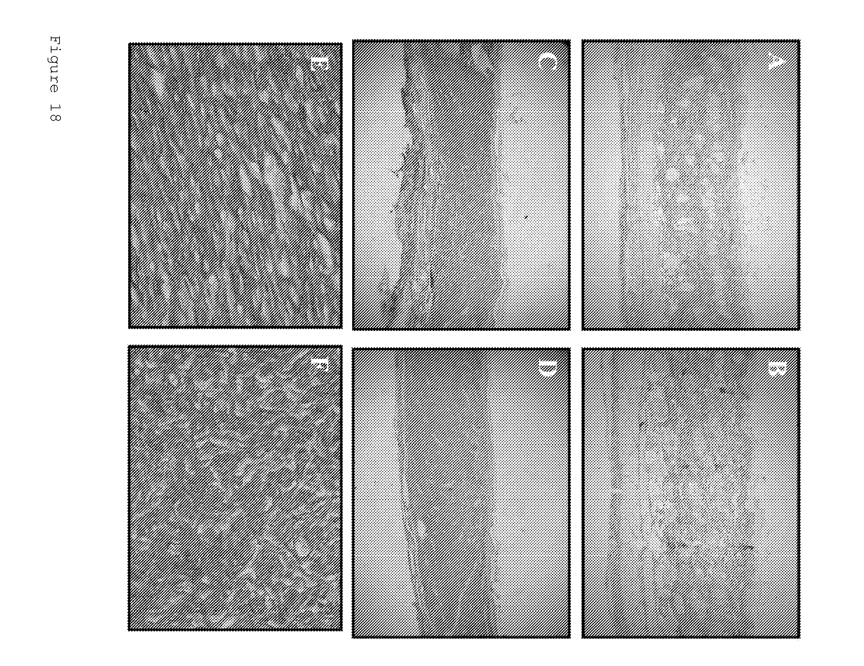

Figure 18 shows microphotographs of 5 µm thick rat skin sections stained with picrosirius red. A and B: intact skin sections of a control rat and a rat subjected to treatment, respectively. C and E: skin wound sections of a control rat (at low and high magnification, respectively). D and F: skin wound sections of rats subjected to treatment with extract (at low and high magnification, respectively).
Figure 19 shows serum levels of C-reactive protein.

### Detailed Description of the Invention

### Extract of an organism of the Lumbricidae family

The authors of the present invention have surprisingly found that the extracts of annelids of the *Lumbricidae* family are capable of accelerating the process of healing of wounds in patients without causing a significant increase of the cell damage response. Thus, in a first aspect, the invention relates to an extract of an annelid of the *Lumbricidae* family.

The term "extract", as used in the present invention, refers to a substance comprising all or part of the active ingredients present in a certain organism. In the specific case of an organism of the *Lumbricidae* family, the extract can contain one or more components coming from the entire organism or from parts thereof such as the prostomium, the trunk, the pygidium, the bristles and other elements of the body.

The term "*Lumbricidae*", as used in the present invention, refers to a family within the *Lumbricina* suborder which includes organisms of the genera *Allolobophora, Allolobophoridella, Aporrectodea, Cernosvitovia, Bimastus, Dendrobaena, Dendrodrilus, Drawida, Eisenia, Eiseniona, Ethnodrilus, Eophila, Eumenescolex, Fitzingeria, Helodrilus, Iberoscolex, Kritodrilus, Lumbricus, Microeophila, Murchieona, Norealidys, Octodriloides, Octodrilus, Octolasion, Octolasium, Perelia, Postandrilus, Proctodrilus, Prosellodrilus, Satchellius* and *Scherotheca.*

In a preferred embodiment the annelid belongs to the genus *Allolobophora.* The genus *Allolobophora* includes species such as *A. altimontana, A. bellicosa, A. caliginosa, A. carpathica, A. chlorotica, A. cryptocystis, A. dubiosa, A. dugesi, A. dugesi dacica, A. foetida, A. fraissei, A. georgii, A. handlirschi, A. japonica, A. leoni, A. longa, A. lozniciana, A. molleri, A. nematogena, A. rosea, A. smaragdina, A. subrubicunda, A. tuberculata, A. turgida* and the like. In a preferred embodiment, the annelid belongs to the species *Allolobophora caliginosa.*

The morphological characteristics of the species *Allolobophora caliginosa* are indicated in Table 1.

**Table 1: Morphological characteristics of Allolobophora caliginosa**

| Family | *Lumbricidae* |
|---|---|
| Species | *Allolobophora caliginosa* f. typica |
| Color | pale pink |
| Length | 40 - 100 mm |
| No. of segments | 120 - 150 |
| Prostomium type | epilobic |
| Segments of the clitellum | 27 - 25 |
| Dorsal pores | 11/12 |
| Type of bristles | in pairs |
| Papules- puberty | 31 - 33 |
| Male pores | 15 |
| Female pores | 14 |
| No. of seminal vesic. | 4 pairs |
| Seg. of seminal vesic. | 9 - 12 |
| Prostate seg. | - |
| No. of spermathecae | - |
| Seg. of spermathecae | 9/10 - 10/11 |
| Seg. of gizzard | 14 and 5 |

### Method for obtaining an extract of the invention

The authors of the present invention have developed a low temperature method for obtaining extracts of annelids of the *Lumbricidae* family. Thus, in another aspect, the invention relates to a method for obtaining an extract of an annelid of the *Lumbricidae* family comprising the steps of
(i) maintaining at least one annelid of the *Lumbricidae* family at a temperature of 20 to 25°C and
(ii) collecting the liquid released by the annelid

The process of extraction according to the present invention requires contacting the annelid at a temperature of 20 to 25°C

In a preferred embodiment, the step is carried out for a time ranging between 2 and 15 minutes counted from the time at which heating at 20-25°C started.

The process of extraction can be carried out by means of the use of superfluids, by means of centrifugation, sonication extraction, reflux extraction or the like. The extraction, if necessary, can be carried out on dry annelids obtained by means of conventional methods such as lyophilization or drying under low pressure.

The extracts obtained according to the method of the present invention can be processed to be enriched in the bioactive components or to eliminate impurities. Additionally, the extract can be in the form of powder by means of drying the extract using conventional drying methods such as drying under low pressure, lyophilization and the like.

In a preferred embodiment, the method of extraction is carried out from annelids belonging to the genus *Allolobophora.* In an even more preferred embodiment, the annelid is of the species *Allolobophora caliginosa.*

In another aspect, the invention relates to an extract obtained by means of the method of the invention.

### Pharmaceutical compositions of the invention

In another aspect, the invention relates to a pharmaceutical composition comprising an extract of the invention and a pharmaceutically acceptable excipient.

As used in the present invention, the expression "pharmaceutical composition" refers to a formulation which has been adapted to administer a predetermined dose of one or several therapeutically useful agents to a cell, a group of cells, an organ, a tissue or an animal. The expression "pharmaceutical composition" also intends to include cosmetic compositions as well as compositions belonging to the so-called gray area between pharmaceutical and cosmetic products, also known as cosmoceuticals.

The compositions of the invention can contain, in addition to the extract of the invention, one or several additional compounds with the capacity to heal wounds such as plant extracts selected from the group of *Equisetum anense, Aloe barbadensis, Amica montana, Amica chamissonis, Symphytum oficinales, Solanum dulcamara, Echinacea pallida, Potentilla erecta, Trigonella foenumgraecum, Juglans regia, Linum usitatissimum, Terminalia sericea, Oenothera biennis, Centella asiatica, Arctium lappa, Capsella bursa-pastoris, Hypericum perforatum, Matricaria recutita, Chamomille recutita, Agrimonia eupatoria, Centaurea cyanus, Larrea tridentata, Populus spec., Echinacea pupurea, Calendula officinalis, Aesculus hippocastanum, Salvia officinalis, Plantago lanceolada, Quercus robot, Glycyrhiza glabra, Quercus petraea, Hamamelis virgian, Cardiospermumhalicacabum, Betuna, Urtica dioica, Buxus chinensis, Lavandula angustifolia, Lavandula hybrida, Crocus sativus, Smilax aspera, Melaleuca alternifolia*, amino acids or *Viola tricolor* or derivatives or salts thereof or mixtures of at least two of the mentioned ones.

The compositions of the invention can also be administered together with skin protective media such as vitamins or the like, glucosamine sulfate, alantoin, biotin, chondroitin sulfate, coenzyme Q10, dexpantenol, honey/honey extract, niacinamide, propolis, vitamin A or esters thereof, vitamin C or esters thereof, vitamin E and esters thereof or derivatives or salts thereof or mixtures of at least two of the mentioned ones.

It is also contemplated that the composition of the invention is incorporated in a tissue adhesive which also contains, for example, fibrinogen and thrombin and, possibly Factor XIII or another plasma coagulation factor to achieve hemostasis. The tissue adhesive can be prepared as a premixture of the composition, fibrinogen and possibly Factor XIII, the thrombin being added to the premixture immediately before applying the tissue adhesive to the wound. Alternatively, the fibrinogen premixture and the composition and, possibly, Factor XIII, can be applied on the wound before the application of thrombin. The thrombin converts *in situ* the fibrinogen into fibrin, thus reproducing the coagulation process occurring naturally in the healing of wounds. The presence of the composition in the tissue adhesive can serve to accelerate the healing process of the wounds as has been discussed previously. A commercial product suitable for including the active substance of enamel is Tisseel^{®}, a two-component fibrin sealant produced by Immuno, AG, Vienna, Austria.

Vitamins, antioxidants, photoprotective media, insect repellents, ether oils, antimicrobial media, wetting agents, perfumes and in particular coenzyme Q10 are preferably chosen as substances for skin care.

The person skilled in the art will appreciate that the nature of the excipient in the pharmaceutical composition of the invention will depend to a great extent on the administration route. In the case of the compositions formulated for their oral (or topical) use, a pharmaceutical composition according to the invention normally contains the composition of the invention mixed with one or more pharmaceutically acceptable excipients. These excipients can be, for example, inert fillers or diluents, such as sucrose, sorbitol, sugar, mannitol, microcrystalline cellulose, starches, including potato starch, calcium carbonate, sodium chloride, lactose, calcium phosphate, calcium sulfate or sodium phosphate; crumbling agents and disintegrants, for example cellulose derivatives, including microcrystalline cellulose, starches, including potato starch, sodium croscarmellose, alginates or alginic acid and chitosans; binding agents, for example sucrose, glucose. sorbitol, acacia, alginic acid, sodium alginate, gelatin, starch, pregelatinized starch, microcrystalline cellulose, aluminum magnesium silicate, sodium carboxymethylcellulose, methylcellulose, hydroxypropyl methylcellulose, ethylcellulose, polyvinylpyrrolidone, polyvinyl acetate or polyethylene glycol, and chitosans; lubricating agents, including glidants and antiadhesive agents, for example magnesium stearate, zinc stearate, stearic acid, silicas, hydrogenated vegetable oils or talc.

In the case of compositions for their percutaneous or topical administration, the composition is formulated, for example, in the form of emulsions, ointments, solutions or dyes, powders, or in other forms, for example in the form of aerosols or foams. Liquid, semisolid and solid pharmaceutical preparations are suitable for the use according to the invention of the compounds mentioned, in particular solutions, creams, ointments, powders and gel preparations, in which the latter is preferably used due to its increased release of the active compound.

In a preferred embodiment, the extracts and the pharmaceutical compositions of the invention are applied topically, i.e., directly to the skin and to other epithelial surfaces of the body.

The pharmaceutical compositions object of the present invention include all types of solid, semisolid and fluid compositions. Compositions of particular relevance are, for example, pastes, ointments, hydrophilic ointments, creams, gels, hydrogels, solutions, emulsions, suspensions, lotions, liniments, shampoos, jellies, soaps, bars, sprays, powders, films, foams, pads, sponges (for example, collagen sponges), pads, dressings (such as, for example, wound absorbent dressings), potions, bandages, plasters and transdermal release systems.

The pharmaceutically acceptable excipients can include solvents, buffering agents, preservatives, wetting agents, chelating agents, antioxidants, stabilizers, emulsifiers, suspending agents, gel-forming agents, bases for ointments, penetration enhancers, perfumes and skin protective agents.

Examples of solvents are, for example, water, alcohols, vegetable or marine oils (for example, edible oils, such as almond oil, castor oil, cocoa butter, coconut oil, maize oil, cotton oil, linseed oil, olive oil, palm oil, peanut oil, poppy oil, rapeseed oil, sesame oil, soy oil, sunflower oil and tea oil), mineral oils, fatty oils, liquid paraffin, polyethylene glycols, propylene glycols, glycerol, liquid polyalkylsiloxanes and mixtures thereof.

Examples of buffering agents are, for example, citric acid, acetic acid, tartaric acid, lactic acid, phosphoric hydrogen acid, diethylamine, etc.

Suitable examples of preservatives for use in the compositions are parabens, such as methyl, ethyl or propyl p-hydroxybenzoate, butylparaben, isobutylparaben, isopropylparaben, potassium sorbate, sorbic acid, benzoic acid, methyl benzoate, phenoxyethanol, bronopol, bronidox, MDM hydantoin, iodopropynyl butylcarbamate, EDTA, benzalkonium chloride and benzyl alcohol, or mixtures of preservatives.

Examples of wetting agents are glycerine, propylene glycol, sorbitol, lactic acid, urea and mixtures thereof. Examples of chelating agents are sodium EDTA and citric acid.

Examples of antioxidants are butylated hydroxyanisole (BHA), ascorbic acid and derivatives thereof, tocopherol and derivatives thereof, cysteine and mixtures thereof.

Examples of emulsifiers are natural gums, for example acacia gum or gum tragacanth; natural phosphatides, for example soy lecithin; sorbitan monooleate derivatives; wool fats; wool alcohols; sorbitan esters; monoglycerides; fatty alcohols; fatty acid esters (for example, fatty acid triglycerides), and mixtures thereof.

Examples of suspending agents are, for example, cellulose and cellulose derivative, such as, for example, carboxymethylcellulose, hydroxyethylcellulose, hydroxypropylcellulose, hydroxypropyl methylcellulose, carrageenan, acacia gum, gum arabic, tragacanth and mixtures thereof.

Examples of gel bases are viscosity-enhancing agents or components capable of collecting exudates from a wound: liquid paraffin, polyethylene, fatty oils, silica or colloidal aluminum, zinc soaps, glycerol, propylene glycol, tragacanth, carboxyvinyl polymers, magnesium and aluminum silicates, Carbopol^{®}, hydrophilic polymers, such as, for example, starch or cellulose derivatives, such as, for example, carboxymethylcellulose, hydroxyethylcellulose and other cellulose derivatives, water-swellable hydrocolloids, carrageenans, hyaluronates (for example, hyaluronate gel possibly containing sodium chloride) and alginates, including propylene glycol alginate.

Examples of ointment bases are, for example, bee wax, paraffin, cetanol, cetyl palmitate, vegetable oils, sorbitan esters and fatty acids (Span), polyethylene glycols and sorbitan ester and fatty acid and ethylene oxide condensation products, for example polyoxyethylene sorbitan monooleate (Tween).

Examples of hydrophobic or water-emulsifying ointment bases are paraffins, vegetable oils, animal fats, synthetic glycerides, waxes, lanoline and liquid polyalkylsiloxanes.

Examples of hydrophilic ointment bases are solid macrogols (polyethylene glycols).

Other examples of ointment bases are triethanolamine soaps, sulfated fatty alcohol and polysorbates.

Examples of powder components are: alginate, collagen, lactose, powder which is capable of forming a gel when it is applied to a wound (it absorbs liquid/exudate of the wound). Normally, the powders intended to be applied in large open wounds must be sterile and the particles present must be micronized.

Examples of other excipients are polymers such as carmellose, carmellose sodium, hydroxypropylmethylcellulose, hydroxyethylcellulose, hydroxypropylcellulose, pectin, xanthan gum, locust bean gum, acacia gum, gelatin, carbomer, emulsifiers such as vitamin E, glyceryl stearates, cetanyl glucoside, collagen, carrageenan, hyaluronates and alginates and chitosans.

In a dental paste or buccal washing formulation or another formulation for applying to the teeth at the dental roots, the composition of the invention can be present in a dissolved state in a carrier with slightly acid pH or as a dispersion in a carrier with neutral pH. It is foreseen that the use of the composition of the invention can form a protective layer on the surface of the teeth.

The aforementioned compositions for topical administration serve more suitably for direct application to the wounds, or they can be suitable to be applied to, or introduced in, relevant orifice(s) of the body, for example the rectal, urethral, vaginal, aural, nasal or oral orifices. The composition can be simply applied directly to the part to be treated, such as, for example, on the mucosa, or by any appropriate administration route.

The compositions which have demonstrated to be important in relation to the topical application are those having thixotropic properties, i.e., the viscosity of the composition is affected, for example, if it is shaken or stirred, such that the viscosity of the composition at the time of administration can be reduced and, when the composition has been applied, the viscosity increases such that the composition remains in the application site.

The compositions suitable for use according to the invention can also be presented in the form of suspensions, emulsions or dispersions. Said compositions contain the active substance of the enamel in mixture with a dispersing or wetting agent, a suspending agent and/or one or more preservatives and other pharmaceutically acceptable excipients. Said compositions can also be suitable for use in administering the composition, for example, to an intact or damaged mucosa, such as the oral, buccal, nasal, rectal or vaginal mucosa, or for administration to the intact or damaged skin or to wounds.

Suitable dispersing or wetting agents are, for example, natural phosphatides, for example lecithin or soy lecithin; the condensation products of ethylene oxide with, for example, a fatty acid, a long chain aliphatic alcohol or a partial ester derived from fatty acids and a hexitol or hexitol anhydride, for example polyoxyethylene stearate, polyoxyethylene sorbitol monooleate, polyoxyethylene sorbitan monooleate, etc.

Suitable suspending agents are, for example, natural gums, such as, for example, acacia gum, xanthan gum or tragacanth gum; celluloses, such as, for example, sodium carboxymethylcellulose, microcrystalline cellulose (for example, Avicel^{®} RC 591, methylcellulose); alginates and chitosans, such as, for example, sodium alginate, etc.

Suitable examples of preservatives for use in compositions according to the invention are the same as those mentioned above.

The compositions for use according to the invention can also be administered by oral route. The suitable oral compositions can be in the form of a particulate formulation or in the form of a solid, semisolid or fluid dosage form.

The compositions for oral use include solid dosage forms, such as, for example, powders, granules, granulates, sachets, tablets, capsules, effervescence tablets, chewable tablets, cachets, immediate release tablets and modified release tablets, as well as fluid or liquid formulations, such as, for example, solutions, suspensions, emulsions, dispersions and mixtures. Moreover, the composition can be in the form of powders, dispersible powders or granules suitable for preparing an aqueous suspension by adding a liquid medium, such as, for example, an aqueous medium.

Other pharmaceutically acceptable excipients can be colorants, flavoring agents, plasticizers, wetting agents, buffering agents, etc.

In those cases in which the pharmaceutical composition is in the solid unit dosage form (for example, a tablet or a capsule), the unit dosage form can be provided with a coating, such as one or more of the coatings mentioned below.

In those cases in which the composition is in the form of a tablet, capsule or multiple unit composition, the composition or the individual units, or a tablet or a capsule containing the individual units, can be coated, for example, with a sugar coating, a film coating (for example, based on hydroxypropylmethylcellulose, methylcellulose, methylhydroxyethylcellulose, hydroxypropylcellulose, carboxymethylcellulose, acrylate copolymers (Eudragit), polyethylene glycols and/or polyvinylpyrrolidone) or an enteric coating (for example, based on methacrylic acid copolymer (Eudragit), cellulose acetate phthalate, hydroxypropylmethylcellulose phthalate, hydroxypropylmethylcellulose acetate succinate, polyvinyl acetate phthalate, lacquer gum and/or ethylcellulose). Moreover, a time delay material such as, for example, glyceryl monostearate or glyceryl distearate, can be used.

For application to the rectal or vaginal mucosa, the suitable compositions according to the invention include (emulsion or suspension type) suppositories, enemas and rectal gelatin capsules (solutions or suspensions). As pharmaceutically acceptable suitable suppository bases cocoa butter, esterified fatty acids, glycerin gelatin and several water-soluble or - dispersible bases, such as polyethylene glycols and polyoxyethylene sorbitan esters and fatty acids, are included. Several additives, such as, for example, enhancers or surfactants can be incorporated.

For application to the nasal mucosa as well as to the oral mucosa, suitable compositions according to the invention are sprays and aerosols for inhalation. In a typical nasal composition, the active substance of the enamel is present in the form of a particulate formulation possibly dispersed in a suitable carrier. The pharmaceutically acceptable carriers and excipients and possibly any other pharmaceutically acceptable material present in the composition, such as diluents, enhancers, flavoring agents, preservatives, etc., are all selected according to conventional pharmaceutical practice in a way that those persons skilled in the art of formulating pharmaceutical products understand.

In a pharmaceutical composition for use according to the invention on skin or mucosa, a composition of the invention is generally present in a concentration of approximately 0.01% to approximately 99.9% w/w. The amount of composition applied will normally result in an amount of total protein per cm² of area of wound/skin/tissue corresponding to approximately 0.01 mg/cm² to approximately 20 mg/cm², such as approximately 0.1 mg/cm² to approximately 15 mg/cm².

The amount of the composition applied depends on the concentration of the composition of the invention and on the release rate of the active substance of the enamel from the composition, but it is generally in a range corresponding to approximately 15-20 mg/cm² at most. In those cases in which the composition of the invention is administered in the form of a fluid composition, the concentration of the active substance of the enamel in the composition is in a range corresponding to approximately 0.1 to approximately 50 mg/ml. Higher concentrations are in some cases desirable and can also be obtained as a concentration of at least approximately 100 mg/ml.

The concentration of the composition of the invention in the pharmaceutical composition depends on the concentration of the composition, on its strength, on the severity of the disease which is to be prevented or treated and on the age and condition of the patient. The methods applied for the selection of relevant concentrations of the composition of the invention in the pharmaceutical composition are well known for a person skilled in the art and can be carried out according to guidelines for Good Clinical Practice (GCP) or Investigational New Drug (IND) Exemption regulations, according to what is described, for example, in the International Standard ISO/DIS 14155 for clinical investigation of medical devices, 1994, and "ICH" (International Conference on Harmonization): harmonized tripartite guideline for Good Clinical Practice, Brookwood Medical Publications, Ltd., Surrey, UK, 1996. A person skilled in the art will be able to, by means of the use of the methods described in standard textbooks, of the guidelines and of the regulations described above, as well as by means of the use of general common knowledge within this field, select the exact dosage regimen to be performed for the composition of the invention and/or select other active substances and dosage forms using only routine experimenting processes.

The pharmaceutical preparations according to the invention are prepared in a way known by itself and familiar to persons with experience in the art and pharmaceutically acceptable inert inorganic and/or organic carriers are used in addition to the compound or compounds of formula I and/or physiological tolerable salts thereof.

The extract of the invention forms part of the pharmaceutical compositions in a percentage ranging between 0.1 to 10.0% by weight, preferably from 0.2 to 5% by weight, with respect to the polymer matrix or 0.001 to 30% by weight, preferably 5 to 15% by weight with respect to the total of the pharmaceutical composition.

### Therapeutic uses of the extract of the invention

In another aspect, the invention relates to an extract according to the invention for its use in the healing of wounds. In another aspect, the invention relates to a method to promote the healing of wounds comprising administering to a subject a composition according to the invention. In another aspect, the invention relates to the use of a composition according to the invention for the preparation of a drug product for the treatment of wounds.

In the present context, the term "wound" relates to a body lesion with a discontinuity of the normal integrity of the tissue structures. The term intends to also encompass the terms "sore", "lesion", "necrosis" and ulcers Normally, the term "sore" is a popular term for almost any lesion of the skin or of the mucosa membranes and the term "ulcer" is a local defect or excavation of the surface of an organ or tissue, produced by the detachment of the necrotic tissue. Lesion generally relates to any tissue defect. Necrosis relates to the dead tissue due to infection, lesion, inflammation or infarctions.

The term "wound" used in the present context means any wound (see below for a classification of wounds) and in any particular phase of the healing process, including the phase before having started any healing or even before a specific wound is produced, such as a surgical incision (prophylactic treatment). Wounds are typically classified in one of four stages depending on the depth of the wound. Thus, stage I wounds are those limited to the epithelium, stage II wounds are those extending to the dermis, stage III wounds are those extending to the subcutaneous tissue and stage IV wounds are those in which the bone is exposed.

The wounds and/or ulcers are normally emerging from the skin or on a mucosa surface or as result of an infarction in an organ ("vascular accident"). A wound can be the result of a defect or a lesion of the soft tissues or of an underlying condition.

In the present context, the term "skin" relates to the outermost surface of the body of an animal, including a human, and encompasses the intact or almost intact skin, as well as a lesioned cutaneous surface. The term "mucosa" relates to a non-damaged or damaged mucosa of an animal, such as a human, and can refer to the oral, buccal, aural, nasal, pulmonary, ocular, gastrointestinal, vaginal or rectal mucosa.

Examples of wounds which can be prevented and/or treated according to the present invention are, for example, open wounds and closed wounds. Open wounds which can be treated with the compositions of the invention include, but are not limited to, burns caused cold or heat, incisions, ulcers, lacerations, abrasions, acne, bite wounds, punctures or gunshot wounds or closed wounds such as contusions or hematomas, lesions of the blood and lymphatic vessels such as Buerger's disease, lymphedema and ulcus cruris, post-surgery wounds such as wounds after a skin transplant and sutured wounds, decubitus ulcer, pressure ulcer, diabetic ulcer, post-herpetic ulcers and lesions by irradiation. Closed wounds which can be treated with the compositions of the invention include, but are not limited to, contusions or hematomas.

Aseptic wounds, contused wounds, incised wounds, lacerated wounds, non-penetrating wounds (i.e., wounds in which there is no discontinuity of the skin, but there is lesion in the underlying structures), open wounds, penetrating wounds, perforating wounds, punctured wounds, septic wounds, subcutaneous wounds, etc can also be treated according to the present invention. Examples of sores are decubitus ulcers, aphthae, chrome ulcers, cold ulcers, pressure ulcers, etc. Examples of ulcers are, for example, peptic ulcer, duodenal ulcer, gastric ulcer, gouty ulcer, diabetic ulcer, hypertensive ischemic ulcer, stasis ulcer, ulcus cruris (venous ulcer), sublingual ulcer, submucosal ulcer, symptomatic ulcer, trophic ulcer, tropical ulcer and venereal ulcer, for example caused by gonorrhea (including urethritis, endocervicitis and proctitis). The conditions related to wounds or sores which can be successfully treated according to the invention are burns, anthrax, tetanus, gaseous gangrene, scarlatina, erysipelas, sycosis barbae, folliculitis, impetigo contagiosa or bullous impetigo, etc. There is frequently an overlapping between the use of the terms "wound" and "ulcer" and "wound" and "sore" and, moreover, the terms are often used randomly. Therefore, as has been previously mentioned, in the present context the term "wound" includes the terms "ulcer", "lesion "', "sore" and "infarction" and the terms are used indistinctly unless otherwise indicated.

The types of wounds to be treated according to the invention also include i) general wounds, such as, for example, surgical, traumatic, infectious, ischemic, thermal, chemical and bullous wounds; ii) specific wounds of the oral cavity, such as, for example, wounds after extractions, endodontic wounds especially in relation to the treatment of cysts and abscesses, ulcers and bacterial, viral or autoimmune lesions, mechanical, chemical, thermal, infectious and lichenoid wounds; herpetic ulcers, aphthous stomatitis, acute necrotizing ulcerative gingivitis and burning mouth syndrome are specific examples; and iii) wounds on the skin, such as, for example, neoplasias, burns (for example, chemical or thermal burns), lesions (bacterial, viral, autoimmune), bite wounds and surgical incisions. Another way to classify the wounds is as i) small loss of tissue due to surgical incisions, minor abrasions and minor bite wounds, or as ii) significant loss of tissue. This latter group includes ischemic ulcers, pressure sores, fistulas, lacerations, severe bite wounds, thermal burns and wounds in donor site (in soft and hard tissues) and infarctions.

In a preferred embodiment the extract of the invention is used for the treatment of a wound in which the wound is selected from the group consisting of an aseptic wound, a contused wound, an incised wound, a lacerated wound, a non-penetrating wound, an open wound, a penetrating wound, a perforating wound, a punctured wound, a septic wound, a subcutaneous wound, an ischemic ulcer, a pressure ulcer, a fistula, a bite wound, a thermal burn wound, a diabetic foot wound and a donor site wound.

As used in the present invention "healing" of a wound refers to the physiological process in which the wounded (damaged) area returns to its normal state. If it refers to an open wound, the healing refers to the process by which the skin or mucosa again forms a continuous barrier by means of the increase of connective tissue and of epithelial cells. The person skilled in the art will appreciate that, after the healing, the wounded area can comprise scar tissue which is not identical to the surrounding tissue. The use of the extract of the invention can prevent or reduce the formation of scar or reduce the unpleasant appearance which scar tissue occurring during the process of healing has.

The contact between the extract of the invention and the damaged tissue can be transitory or non-transitory, i.e., it is maintained for a substantial time period. For example, the treatment with the extract of the invention can be carried out for, but is not limited to, 10 minutes or more, an hour or more, preferably at least 2, at least 3 or at least 4 hours.

### Cosmetic uses of the extract of the invention

The extract of the present invention has shown to be useful in the treatment of non-pathological conditions in which it is necessary to increase the tissue repair in the skin or mucosa of an individual. Thus, in another aspect, the invention relates to a method for cosmetic treatment comprising administering an extract according to the invention.

"Cosmetic treatment", as used in the present invention, means any treatment improving the physical appearance or the odor of a subject.

The defects that can be treated cosmetically using the extract of the invention include, but are not limited to, non-esthetic skin, defects in the skin such as acne, freckles, cellulitis, scars, premature skin ageing caused by exposure to the sun or ultraviolet radiation, defects in the skin associated to ageing such as age spots, wrinkles, stretching, signs of ageing and the like.

The cosmetic use of the extract of the invention is essentially carried out by means of the direct topical application to the skin or to the mucosae.

Preferably, the use of the extract of the invention in a cosmetic method requires its formulation as a patch, a capsule, a cream, a detergent, a shampoo, a soap or dermosoap, bath salts, an aqueous, alcohol or oily body lotion, an aerosol, a gel, an emulsion, a lipstick, nail polish, manicures, shaving foam, a deodorant, an aftershave and the like. Alternatively, the extract of the invention is formulated in the form of liposomes or of any other type of formulation which allows a sustained release of the extract. The cosmetic formulations comprise, in addition to the extract of the invention, one or more additional active ingredients and one or more cosmetically acceptable excipients.

The expression "cosmetically acceptable excipient", as used herein, refers to an excipient which can be used in different parts of the body without causing toxicity, irritation, allergy or any other undesired reaction. Excipients suitable for their use in the present invention include, but are not limited to, fragrances, perfumes, emollients, antiseptics, pigments, colorants, wetting agents, propellants, film formers, vitamins and other types of materials the presence of which can be interesting from the cosmetic, dermatological, pharmaceutical point of view. Examples of this type of agent can be found in CTFA International Cosmetic Ingredient Dictionary 12th Edition, The Cosmetic, Toiletry, and Fragrance Association, Inc., Washington, D.C., 2004.

### Materials comprising the extract of the invention

In another aspect, the invention relates to a material for treating a wound comprising an extract of the invention. The term "comprises", as used herein, includes materials either coated or impregnated with the extract of the invention. The material can be woven (for example, a fabric) or non-woven. Suitable materials include, but are not limited to, medical bandages such as sticking plasters, gauzes, dressings and the like.

When dressings are used as a dosage form, the active substance according to the invention can be mixed with other materials/ingredients before or during the manufacture of the dressing, or the composition of the invention can be deposited on the dressing in some manner, for example immersing the dressing in a solution or dispersion of the composition or spraying a composition on the dressing. Alternatively, the composition of the invention can be applied to the dressing in the form of powder. The dressings can be in the form of wound absorbent dressings for application to exudative wounds. The dressings can also be in the form of hydrogel dressings (for example, cross-linked polymers, such as, for example, Intrasite^{®}, containing carboxymethylcellulose, propylene glycol or polysaccharide, disaccharide and proteins), in the form of occlusive dressings, such as, for example, alginates, chitosan, hydrophilic polyurethane film, collagen laminae, sheets, powders, foams or sponges, foams (for example, polyurethane or silicone), hydrocolloids (for example, carboxymethylcellulose, C1VIC), collagen and dressings based on hyaluronic acid, including combinations thereof.

Dressings of alginate, chitosan and hydrocolloid collect the exudate of the wound when they are placed on a wound. Upon doing this, an aqueous gel is produced on the surface of the wound and it is believed that this gel favors the healing of the wound due to the retention of moisture in the wound.

Suitable bandages include, but are not limited to:
- Alginate bandages are indicated for highly exudative wounds, since they have the capacity of absorbing 20 times their weight.
- Generally multilayer composite bandages incorporating a non-adherent layer which is in contact with the wound, and several absorbent, draining and semi-occlusive layers. The absorbent layer does not incorporate a foam, alginate, hydrocolloid or hydrogel. A support of non-woven adhesive tape secures the bandage to the skin. These bandages are used as covering bandages for all the phases of the healing of wounds and are primary bandages for many types of post-operatory wounds.
- Foam bandages, preferably made of polyurethane, although silicone is also used.
- 100% cotton thread gauze, including both woven and non-woven gauze
- Hydrocolloid bandages
- Hydrogel bandages
- Transparent film bandages
- Biological and biosynthetic bandages such as bandages based on collagen, bandages built from related biocompatible amino acids and protein hydrolysate and bandages which combine synthetically and biologically derived materials.

The extract of the invention can be topically administered to human beings and other mammals such as dogs, cats, horses, pigs, calves or sheep.

The pharmaceutical preparations normally contain from approximately 0.05% to approximately 5%, preferably from 0.5% to 2%, especially preferably 1%, by weight of the extract of the invention. Lower and higher percentages are also possible.

In addition to the extracts of the invention, the pharmaceutical compositions of the invention can contain additives such as, for example, fillers, linking agents, lubricants, wetting agents, stabilizers, emulsifiers, preservatives, colorants, thickening agents, diluents, buffering substances, solvents, solubilizing agents, agents to achieve an extended effect, salts to alter the osmotic pressure, covering agents or antioxidants.

Additionally, the compositions of the invention are useful for the treatment of wounds including those produced by burns by cold, heat, surgery, diseases having a delay in the healing of wounds as well as the cosmetic treatment of the wounds as a result of the skin stretching and the protection against the radiation and oxidant agents.

The invention is illustrated below based on the following examples provided by way of a non-limiting illustration of the scope of the invention .

### Examples

### Example 1

### PREPARATION OF THE ALLOLOBOPHRA CALIGINOSA EXTRACT

1- 300 to 2000 individuals (annelids) the length of which is greater than 3 cm and less than 6 cm are selected manually from a breeding place.
2- They must be in conditions of 80%-85% humidity and vivacious state.
3- They must not have oothecae in their body while they are collected.
4- They are joined in an approximate number of 500 annelids in open plastic containers for about two hours such that they eliminate dirt or foreign elements between them.
5- The temperature at the extraction time must not be greater than 15° and the pH greater than 8.
6- They are subsequently washed in larger vessels, taking care to clean all the dirt remaining from the breeding place. An over-absorption of water in their body is also avoided during this process. To that end, such process must not last more than 10 minutes for every wash.
7- The water used must only be rural drinking water, deep wells with high mineral content.
8- They are subsequently left for about 20 minutes in plastic containers (pots) in a number of 500 so that they eliminate part of the water absorbed during the washing process and raise their body temperature.
9- They are then placed in an aluminium pan-type plate on an electric stove at a temperature between 20 to 25° Celsius for about 15 minutes, in which process the liquid which the annelids gradually lose is collected from minute two to fifteen.
10- The liquid is collected in 20 to 30 ml containers and they are left open for 3 minutes before sealing it.
11- The process vessel: plate and pan are washed with abundant chlorine-free water and the same process is carried out again each time extract is extracted.
12- The process conditions are sterile and the following must be used: gloves, mask, head cap, booties. All these elements change each time for the extraction of samples.
13- The samples are stored in a fridge at a temperature of 7° until they are used or sent for analysis.

### Example 2

### MATERIALS AND METHODS

### 2.1. Animals

Male Sprague-Dawley rats with a weight of approximately 250 grams obtained from the animal care facility of a locally prestigious university were used in this study. During the study, the animals were kept in polypropylene cages in controlled conditions of light and temperature and fed with water and normal food (pellets) *ad libitum*, according to the "Guide for the care and use of laboratory animals" published by the "National Health Institute" (NIH No. 85-23, 1985).

### 2.2. Surgical wounds by incision

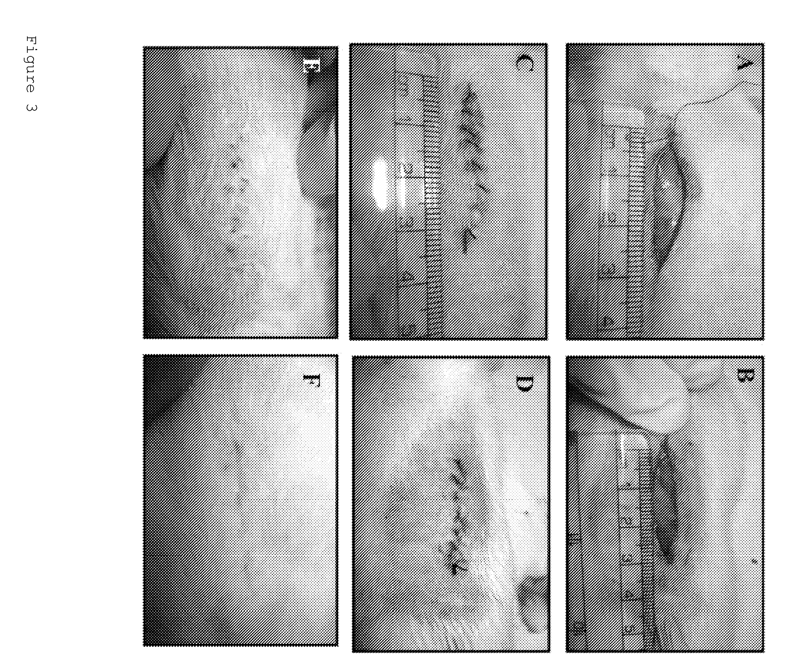

The model of wound by incision of the skin according to the standard procedure previously described in the literature (Sandberg, 1964; Ehrlich, 1968; Nayak, 2006) was used. Briefly, before the procedure the animals were anesthetized with an intraperitoneal injection of ketamine (50 mg/kg) and xylazine (10 mg/kg). After being anesthetized, the skin of the back was shaved with an electric shaver, the animals were taken to the surgery table and the skin was disinfected with 70% ethanol. Using surgical scissors, tweezers and a scalpel, a 3 cm long wound by incision was made on the dorsal midline at 1 cm from the occiput, covering the thickness of the skin until the panniculus adiposus (Figure 3). The skin was then closed using a 4.0 silk suture and the animals were left in recovery. According to the protocol, the animals were randomly distributed into two groups which received the topical application of the substance or physiological saline on the area of the wound once a day for a follow-up period of 10 days. Together with the clinical observation, photographs were taken at the end of the surgical procedure and then daily prior to the application of the extract in order to document the evolution of the wounds.

### 2.3. Surgical wound by excision

This procedure was performed according to what has been previously reported in the literature (Shetty et al., 2006, Int J Low Extrem Wounds 5:137-143). Briefly, the animals were anesthetized with an intraperitoneal injection of ketamine (50 mg/kg) and xylazine (10 mg/kg). After being anesthetized, the skin of the back was shaved with an electric shaver, the animals were taken to the surgery table and the skin was disinfected with 70% ethanol and an area of skin of approximately 5 cm², which was cut and extracted, was marked. Immediately afterwards, the extracted area was measured and physiological saline or extract was applied according to the experimental group in question. Once recovered, the animals were returned to their respective cages. Physiological saline or extract, as appropriate, was applied daily and measurements of the area of the scar and photographs were taken every 3 days. The animals were sacrificed four weeks after performing the procedure.

### 2.4. Sacrifice and collection of samples

At the end of the follow-up period, the animals were sacrificed by decapitation and skin and blood samples were taken for their subsequent analysis. Both in the experimental group subjected to wounds by incision and by excision the skin of the wound was extracted entirely, its length was then measured and it was submersed in 4% formalin for its fixing and subsequent inclusion in paraffin.

### 2.5. Histology

The inclusion in paraffin and sectioning of the skin samples were performed by an operator blind to the procedure. Five µm thick histological sections stained with hematoxylin and eosin were used for the light microscope analysis. Microphotographs of the histological sections from all the animals were taken. The histological analysis was performed by an anatomopathologist blind to the procedure.

### 2.6. Immunohistochemistry for Ki67

This technique was implemented to study the cell proliferation of the epidermis. To that end, the skin segments fixed in 4% formalin were dehydrated at room temperature and embedded in paraffin to then obtain 5 µm thick sections. For the identification of proliferating cells the sections were immunostained with antibodies targeted against the nuclear antigen Ki67. After being deparaffinized and after blocking tissue peroxidase with 15% H2O2, antigen retrieval was performed by heating in a microwave with 1 mM EDTA. They were subsequently again blocked with H2O2, washed with PBS-0.05% Tween 20 and incubated with anti-Ki67 antibody (TEC-3, Dako) diluted in a 1/25 proportion in PBS-0.05% Tween 20 + 1% BSA, overnight at 4°C. Biotinylated antibodies labeled with the streptavidin-peroxidase detection system were used for the detection reaction. The sections were counterstained with hematoxylin. For the analysis of proliferating cells, Ki67-positive cells were counted from microphotographs taken from 16 fields of vision per epidermal tissue, which covered intact areas and wounds in a pre-established and identical pattern for all the sections studied. The cells were stained, photographed and counted by a blind operator who did not know the experimental group from which the samples came.

### 2.7. Measurement of serum levels of C-reactive protein

For the purpose of determining if the exposure to the extract and the different surgical procedures to which the animals were subjected cause a specific inflammatory reaction, the levels of C-reactive protein in the serum of animals was measured according to what is described by Diaz Padilla et al. (Immunology, 2003, 109:564-571). Briefly, the serum was obtained by centrifuging the blood at 7000 g for 10 minutes and the supernatant, free of prominent elements, was frozen at -80°C until its use. The levels of C-reactive protein were measured by a commercial capture enzyme-linked immunosorbent assay (ELISA), for which 100 µl of a 1:12000 dilution of each one of the sera were added in the corresponding wells and incubated for 30 minutes at room temperature. After eliminating the serum, each of the wells was washed 5 times with PBS-0.05% Tween 20, and 100 µl of anti-rat peroxidase-conjugated C-reactive protein antibody were subsequently added. After 30 minutes of incubation, 5 washes identical to those described previously were performed, and 100 µl of trimethylbenzidine substrate were added per well. It was incubated in darkness for 10 minutes and 100 µl of phosphoric acid stop solution were added. Finally, the absorbance of each well was measured in an ELISA reader at 450 nm.

### 2.8. Morphometric analysis of collagen

For the purpose of evaluating the amount and quality of the collagen present in the skin of the experimental animals, 5 µm thick histological skin sections were stained with picrosirius red. Epidermis and dermis images were captured with a digital camera coupled to the microscope with a 20X magnification. The images were subsequently processed digitally to calculate the fraction of dermal collagen by using a program previously created in the laboratory with the MATLAB platform (Ocaranza et *al.,* 2002). The photographs were taken and the analysis was performed by blind observers, i.e., observers who did not known the experimental group from which the samples came.

### 2.9. Statistical analysis

The results are expressed as the average ± the standard error of the mean (SEM). The differences between the experimental groups were evaluated by means of the non-parametric Mann Whitney test. A significance level of p<0.05, i.e., the accepted probability that the differences possibly observed in the different experiments are random is equal to or less than 5%, was used.

### Example 3

### ANIMALS SUBJECTED TO SKIN WOUNDS BY INCISION

### 3.1. Clinical evolution of the animals and the wounds

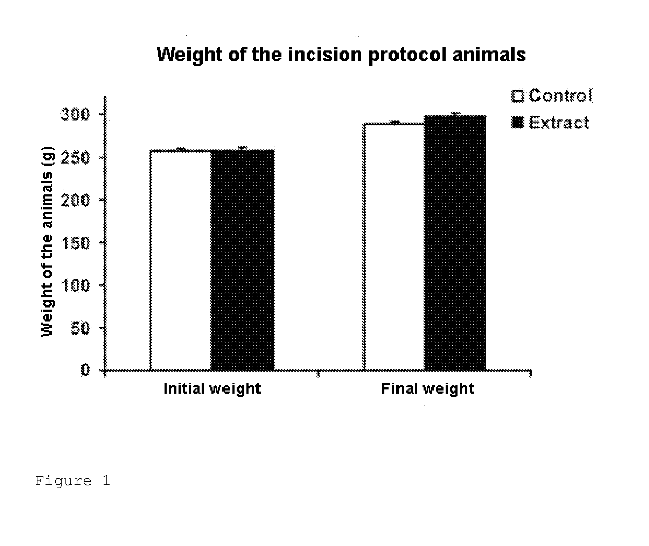

From a total of 24 male rats of 250 grams which were subjected to the incision protocol, 12 were treated with extract and the remaining ones with physiological saline, forming the control group. There were no deaths or morbid processes - infections, tissue necrosis, tumor formations or others in any of the groups considered, confirming the correct performance of the procedure and the innocuousness of the organic extract. As shown in Table 1 and Figure 1, there were no statistically significant differences in the weight of the animals at the time of the sacrifice, which suggests that the extract does not cause negative systemic effects, at least in the evaluated conditions.

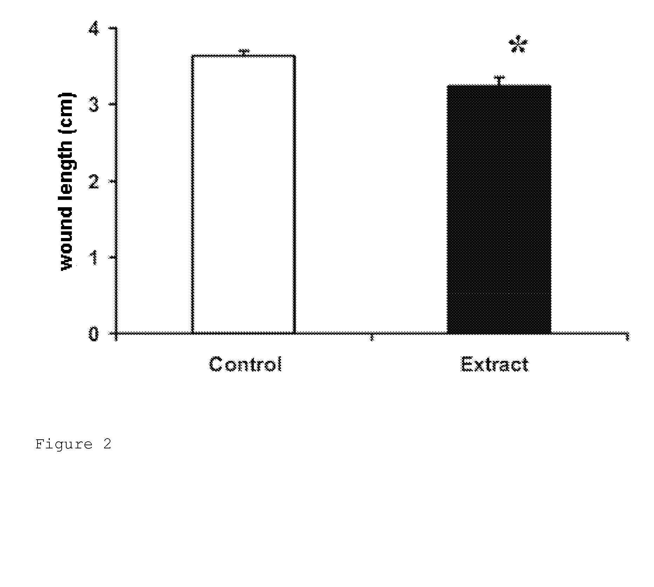

The clinical evolution of the animals was evaluated and photographed daily, prior to the administration of the extract or the physiological saline, as appropriate. Early on, the lower tumor formation of the wounds of the animals treated with extract (i.e., the lower volume and swelling of the wounds) compared to the wounds of the control rats was striking. A neater scar with fewer clinical signs of inflammation i.e., with less reddening of the edges of the wounds was additionally observed. Figure 3 shows representative photographs of what is observed. These phenomena tended to become more pronounced as the days passed, and were maintained until the time of the sacrifice of the animals, carried out 10 days after starting the protocol, according to that published previously in the literature (Nayak, 2006, BMC Complement Altern Med 6, 12). Interestingly enough, the final length of the wounds was shorter (approximately 11%) in the rats treated with the extract than in the animals treated with physiological saline, a difference which was statistically significant (Table 1 and Figure 2).

**Table 1: Animals included in the incision protocol. The results are shown as average ± standard error of the sample. ns = non-significant (p value>0.05), Mann-Whitney test.**

| Animals included in the incision protocol | | Control group | Extract group | p value |
|---|---|---|---|---|
| N | | 12 | 12 | |
| Weight (g) | | | | |
| | Initial | 257±2.5 | 257±4.6 | ns |
| | Final | 289±2.1 | 297±4.9 | ns |
| Length of the wound (cm) | | | | |
| | Initial | 3±0.1 | 3±0.1 | ns |
| | Final | 3.6±0.1 | 3.2±0.1 | p<0.05 |
| Ki-67 cells in epidermis | | | | |
| (cells/field) | | | | |
| | Wound area | 43±10 | 55±13 | ns |
| | | | | |
| | Healthy area | 24±6 | 25±5 | ns |
| | | | | |
| Morphometry of collagen (%): | | | | |
| | Wounded area | 27.5±3.7 | 29.8±3.6 | ns |
| | Healthy area | 31.5±2.3 | 31.3±2.2 | ns |
| Serum levels of CRP | | 27.4±4.9 | 19.3±4.1 | ns |
| (ng/ml) | | | | |

### 3.2. Histology

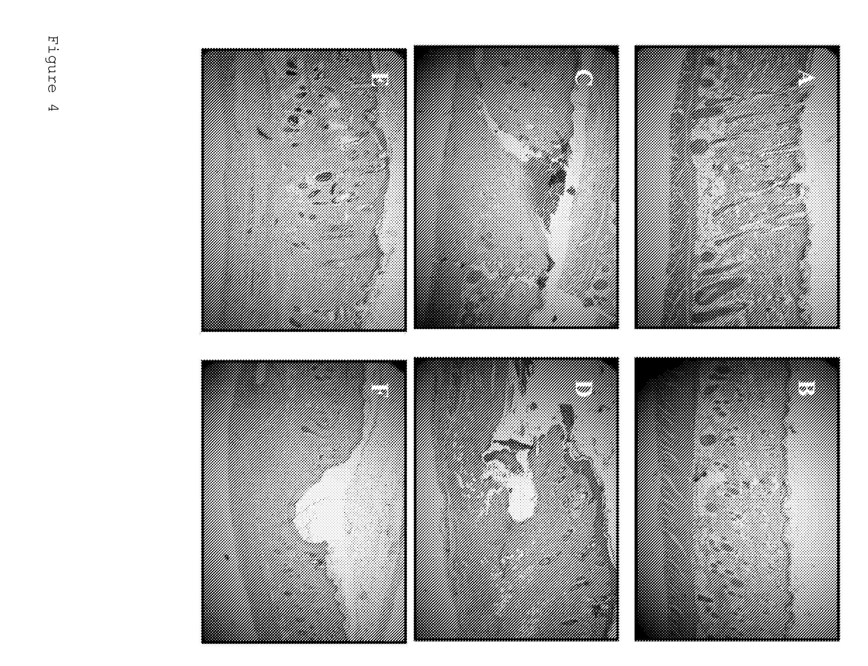

For the purpose of determining the effect of the extract on the healing of wounds by incision, a histopathological analysis of the skin sections stained with hematoxylin and eosin was performed (Figure 4). Generally, the architecture of the tissues is equally conserved in both experimental groups. Both in the group of interest and in the control group the wounds were completely re-epithelialized (the epidermal tissue was restored), without observing differences in the degree of acute inflammation, evaluated by the presence of inflammatory infiltrate, edema and capillary engorgement. There was no considerable presence of granulation tissue or signs of a chronic inflammatory process, measured by the presence of monocytes, macrophages and lymphocyte infiltrate. These signs together form the typical morphology of a mature scar tissue. There were also no differences in the degree of regeneration and thickness of the panniculus carnosus; differences were only observed in the degree of conservation of the skin annexes, measured by the degree of the indemnity and development of sweat glands, sebaceous glands and hair follicles, the latter being greater in the group of animals which received the extract.

### 3.3. Immunohistochemistry for Ki67

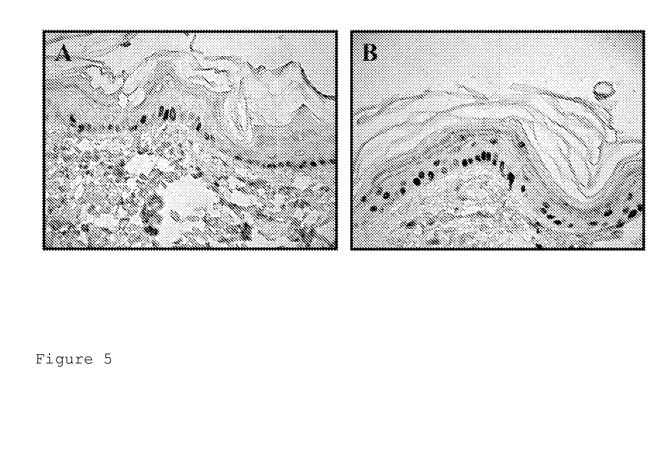

Ki67 is a nuclear protein specific for replicating cells, i.e., it is only present in the nucleus of cells which are dividing, therefore their detection gives an idea of the cell multiplication occurring in a certain tissue at a specific time. Bearing in mind the favorable clinical evolution observed in the wounds of rats treated with the extract, and the early period in which they occurred, the decision was made to study whether at least part of this effect was the result of a greater replicative activity of the cells of the basal layer of the epidermis. To that end, the technique of immunohistochemistry for Ki67, usually used to determine the number of replicating cells in a tissue, was implemented (Figure 5).

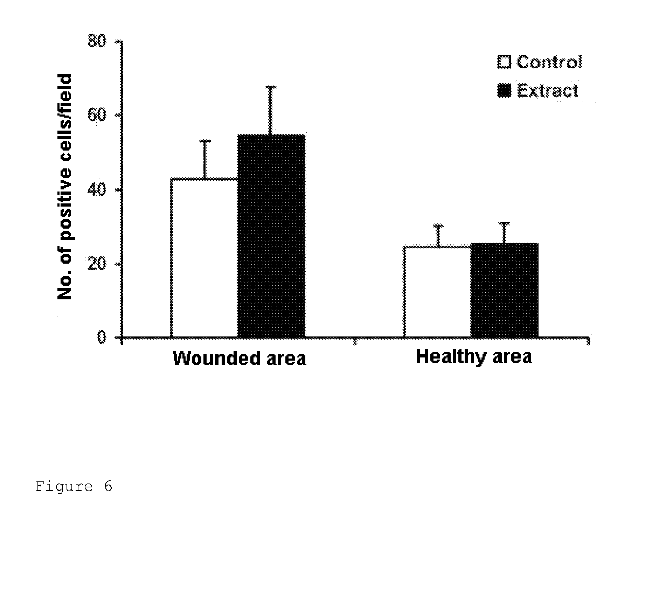

As shown in Figure 6, it is very positive for clear differences to be observed in the labeling of the antigen in skin samples of rats of both groups. Upon quantifying these results, statistically significant differences were observed between the considered groups, which can be interpreted as -at least in the studied conditions. Therefore, based on the results obtained in this study it can be concluded that there was greater cell multiplication in the epidermis of rats treated with extract compared to the epidermis of the control animals.

### 3.4. Morphometry of collagen

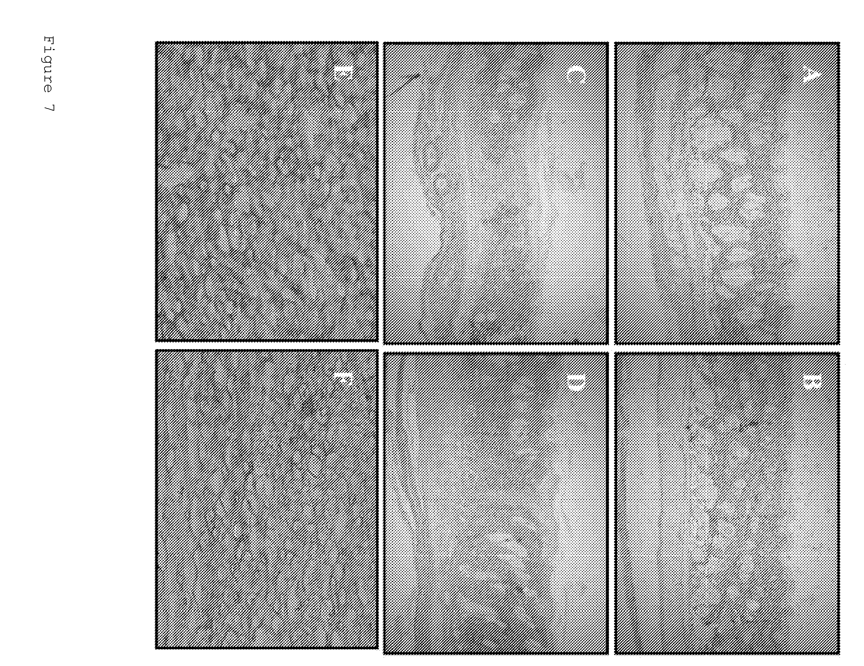

As mentioned previously, the biological repair process is characterized by the substitution of one tissue with another, in the case of the wounds, with collagen. The latter is a group of proteins present in scar tissue, on the deposition of which the strength and the resistance of the scar depend. For the purpose of evaluating whether the application of the extract has an effect on the accumulation of collagen present in the dermis, 5 µm thick cross-sections were taken of the skin wounds of the animals, which were subsequently stained with picrosirius red, a specific agent for detecting collagen. The sections were viewed under a light microscope and microphotographs (see Figure 7) were taken, which were then analyzed by means of an application with support in the MATLAB 6.1 software.

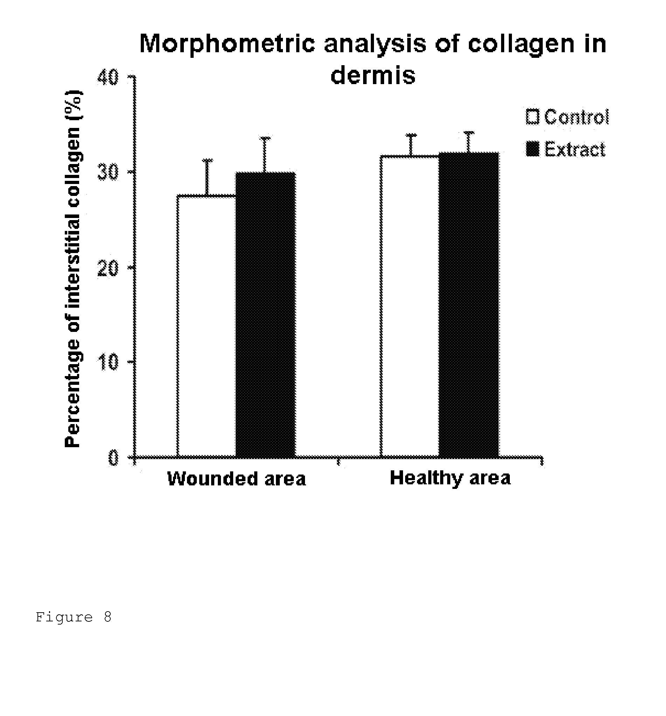

As can be seen in Figure 8, the sections taken from the control animals and the animals treated with the extract, there are differences in the results, according to the graph, on the morphological analysis of the wound, after applying the extract.

### 3.5. Serum levels of C-reactive protein

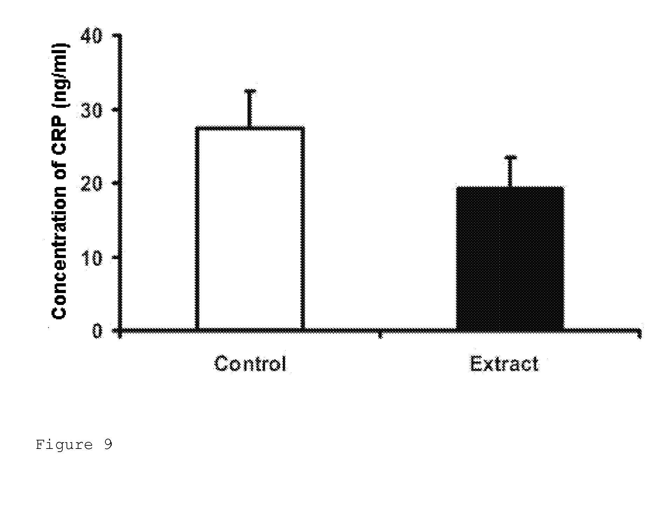

Given the surgical procedure to which the rats were subjected, and considering the fact that part of them received an unprocessed organic extract (i.e., without the addition of preservatives, excipients or other substances which are usually added to this type of product), the decision was made to measure the levels of the C-reactive protein in the serum of the animals at the time of the sacrifice. This peptide belongs to the family of acute-phase proteins, it is synthesized in the liver and rises in response to different stress stimuli, such as severe wounds, traumas, burns, inflammatory processes and infections. Due to this response profile, the C-reactive protein is a valuable -although indirect- indicator of stress in human and veterinary medicine (Diaz-Padilla et al., 2003, Immunology 2003; 109: 564-571). As can be seen in Figure 9, there is a significant difference in favor of the application of the extract, i.e., according to the graph: the C-reactive protein has decreased and the application of the extract has achieved reducing the inflammation.

### Example 4

### ANIMALS SUBJECTED TO SKIN WOUNDS BY EXCISION

### 4.1. Clinical evolution of the animals and the wounds

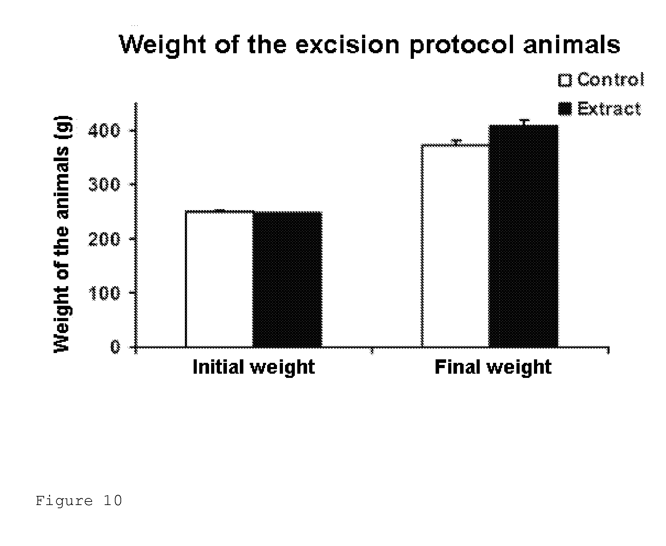

Like in the previous protocol, a total of 24 male rats of 250 grams were subjected to the procedure, which consisted of the resection of an area of 5 cm² of skin in the back of the trunk. Half of them were treated daily with extract and the rest with physiological saline and periodic measurements and photographs were taken of the wounds. Clinically, the animals had a good general condition, without signs of infection or of systemic involvement, which was consistent with what was observed in the animals subjected to wounds by incision. No differences were observed in the initial weights of the rats, and the difference of weight at the end at the time of the sacrifice can be considered irrelevant, in the groups treated with extract or with physiological saline, or in the weights at the time of the sacrifice (Figure 10 and Table 2).

**Table 2: Animals included in the excision protocol**

| | | Control group | Extract group | p value |
|---|---|---|---|---|
| N | | | | |
| Weight (g) | | | | |
| | Initial | 249 ± 2.0 | 274 ± 1.5 | ns |
| | Final | 373 ± 7.9 | 409 ± 9.9 | p<0.05 |
| Wound area (cm²) | | | | |
| | Day 0 | 6.66 ± 0.18 | 6.59 ± 0.28 | ns |
| | Day 3 | 4.87 ± 0.24 | 3.93 ± 0.29 | p<0.05 |
| | Day 6 | 2.67 ± 0.18 | 2.19 ± 0.27 | p<0.05 |
| | Day 9 | 1.31 ± 0.10 | 0.94 ± 0.09 | p<0.05 |
| | Day 12 | 0.74 ± 0.05 | 0.55 ± 0.04 | p<0.05 |
| | Day 15 | 0.50 ± 0.05 | 0.29 ± 0.03 | p<0.05 |
| | Day 18 | 0.29 ± 0.03 | 0.14 ± 0.02 | p<0.05 |
| | Day 21 | 0.20 ± 0.04 | 0.07 ± 0.03 | p<0.05 |
| | Day 24 | 0.09 ± 0.02 | 0.02 ± 0.01 | p<0.05 |
| | Day 27 | 0.03 ± 0.01 | 0.00 ± 0.00 | p<0.05 |
| | Day 30 | 0.01 ± 0.01 | 0.00 ± 0.00 | p<0.05 |
| Day of closure of | | 27.1 ± 1.0 | 22.6 ± 0.8 | p<0.05 |
| the wound (day) | | | | |
| Morphometry of | | | | |
| collagen (%) | | | | |
| | Wound area | 43.0 ± 1.7 | 41.7 ± 1.6 | ns |
| | Healthy area | 30.4 ± 2.4 | 30.8 ± 2.1 | ns |
| Serum levels of CRP | | 17.4 ± 2.2 | 13.4 ± 1.3 | ns |
| (ng/ml) | | | | |

| | | | | |
|---|---|---|---|---|
| The results are shown as average ± standard error of the sample. ns = non-significant (p value>0.05), Mann-Whitney test. | | | | |

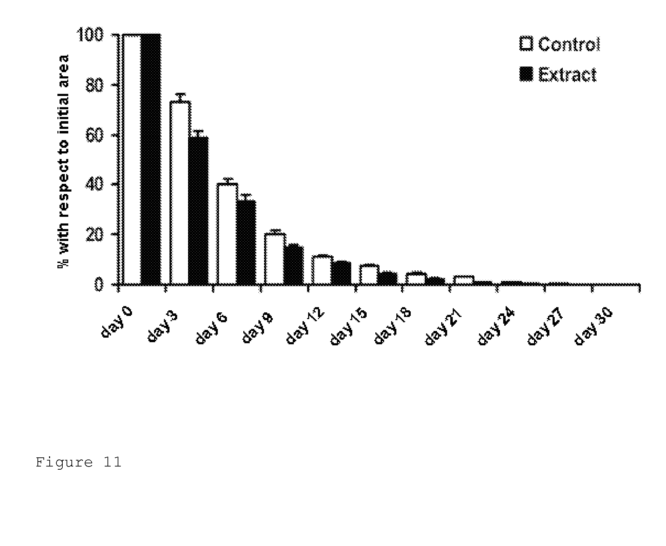

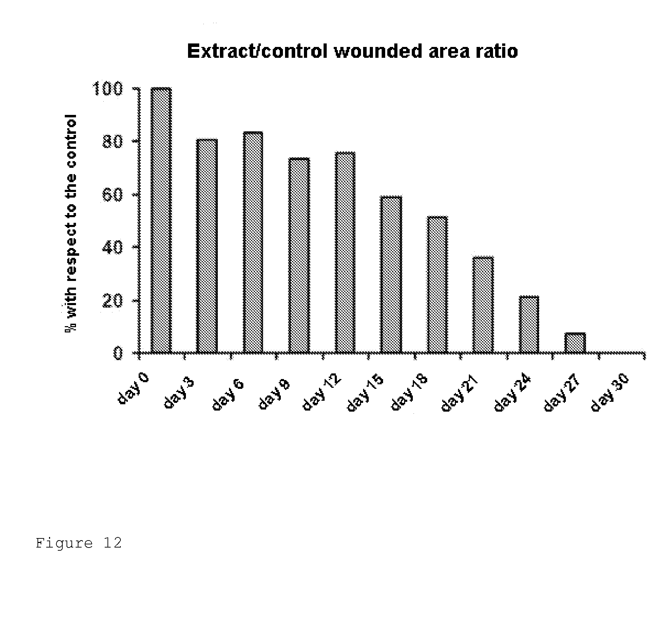

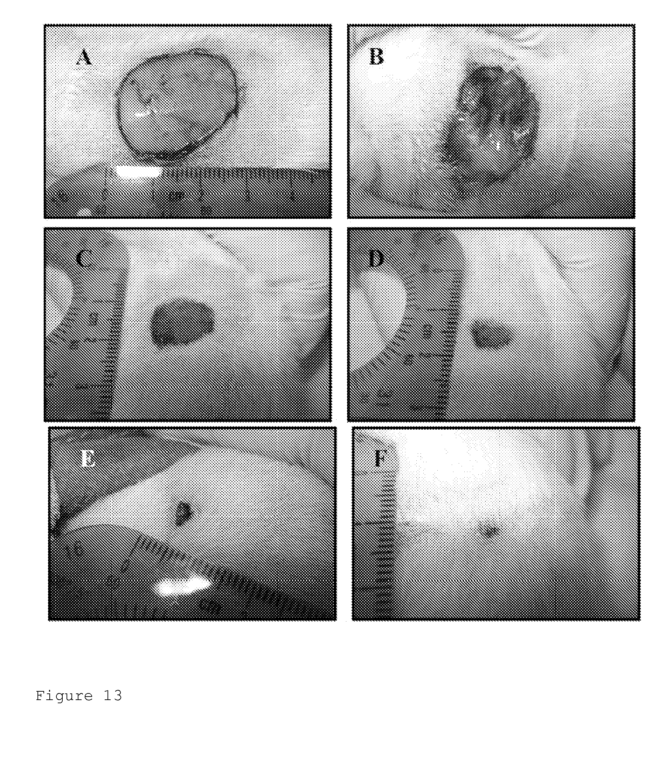

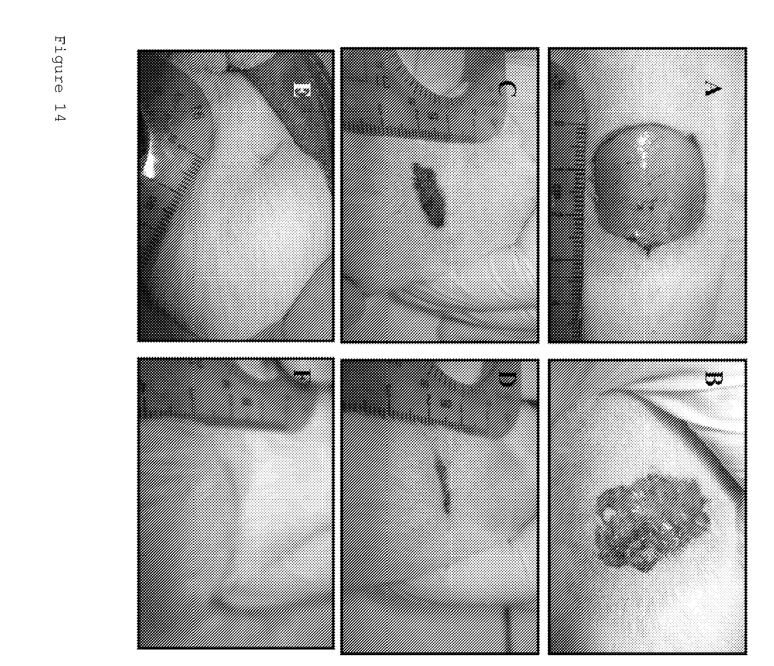

A clear difference was observed early on in the evolution of the wounds treated with extract; thus, already on day 3 after surgery the wounds of the animals treated with extract had an area 20% smaller than the wounds of animals treated with physiological saline, this difference being statistically significant. On day 9 this difference was 27%, on day 15 it was 41% and on day 18 it was 47%, all of them being statistically significant (Figures 11 and 12). Interestingly enough, and as shown in Figures 13 and 14, the reduction of the size was mainly due to a reduction of the crown-rump diameter of the wounds; this finding will be discussed in the conclusions section.

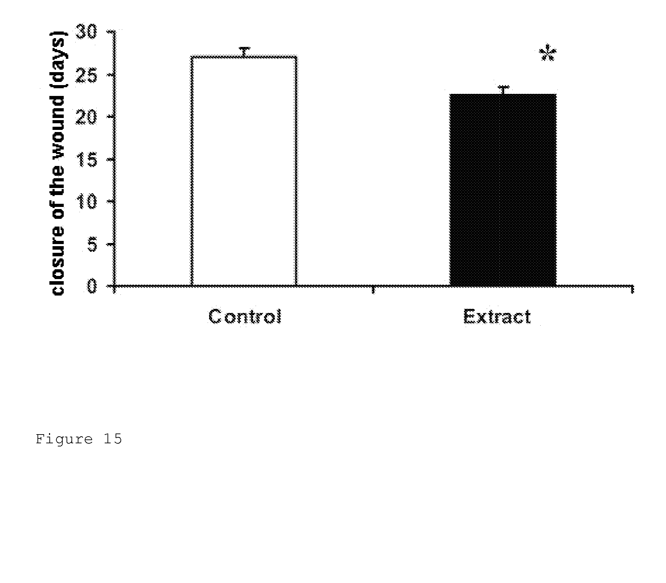

With respect to the closure day, i.e., the day on which an indemnity and continuity of the skin, without the presence of eschar (scabs) was already observed, the wounds of rats treated with extract had consistent tendency to close sooner compared to the wounds of rats treated with physiological saline. The average closure day was 27 for the control animals and 22 in those treated with extract, the difference being approximately 5 days, which was statistically significant (Figure 15).

### 4.2. Histology

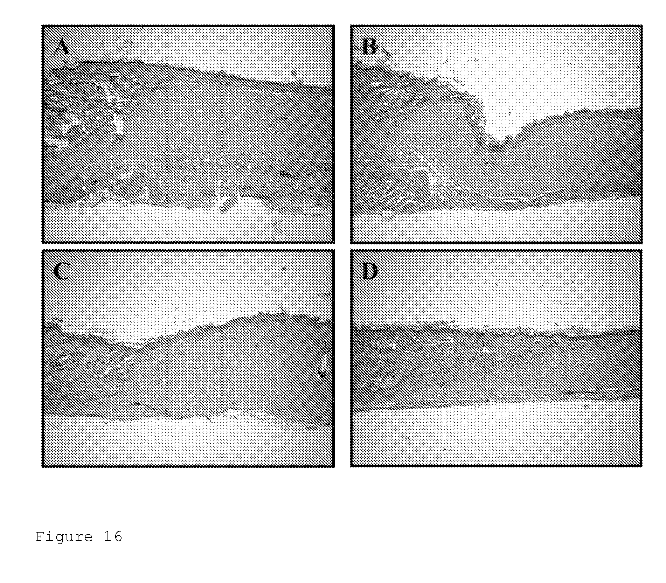

For the purpose of determining the effect of the extract on the healing of wounds by excision, a histopathological analysis of the skin sections stained with hematoxylin and eosin was performed (Figure 16). Generally, a completely re-epithelialized scar in an advanced healing condition was found in both experimental groups. Little or no presence of acute inflammatory elements (polymorphonuclear infiltrate, edema or young granulation tissue) was observed. A greater presence of chronic inflammation elements was observed in the group of control animals with respect to those treated with extract, the latter being characterized in lymphocyte infiltrates, macrophages and considerable presence of giant cells forming inflammatory tissue foci under the dermis. In both groups of animals the panniculus carnosus is absent in large areas under the area of the wound, having a greater degree of regeneration in the group of interest with respect to the control, as well as also in the degree of conservation of the skin annexes, evaluated by the indemnity and development of the sweat glands, sebaceous glands and hair follicles. In summary, in the studied model the application of the extract was associated to a lower presence of chronic inflammatory foci and to a greater degree of regeneration and conservation of the skin annexes and the muscle layer.

### 4.3. Morphometry of collagen

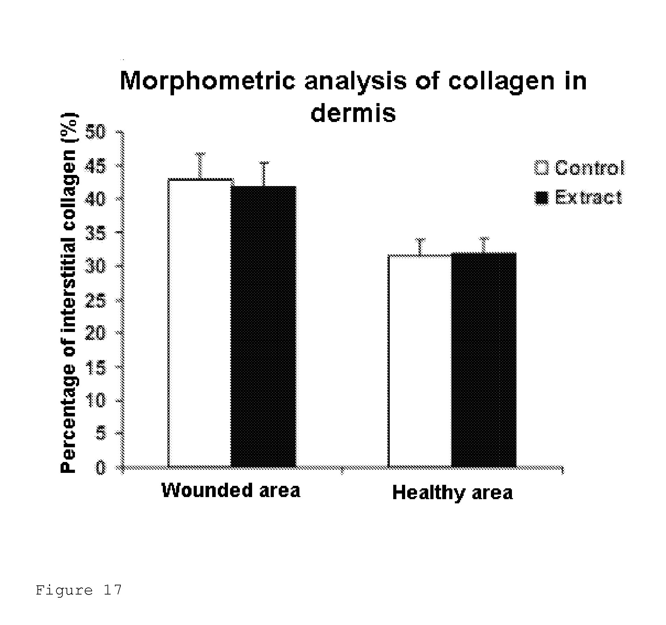

Using the same procedure performed with the incision group, the content of interstitial collagen in the skin samples was measured using morphometry with picrosirius red. As can be seen in Figure 17, the sections taken from control animals and animals treated with the extract did not have clear variations in the amount or in the arrangement of dermal collagen, and the quantification of the phenomenon did not show statistically significant differences (see Figure 17). This means that the application of the extract did not cause changes in the content of collagen present in the skin of the rats.

### 4.4. Serum levels of C-reactive protein

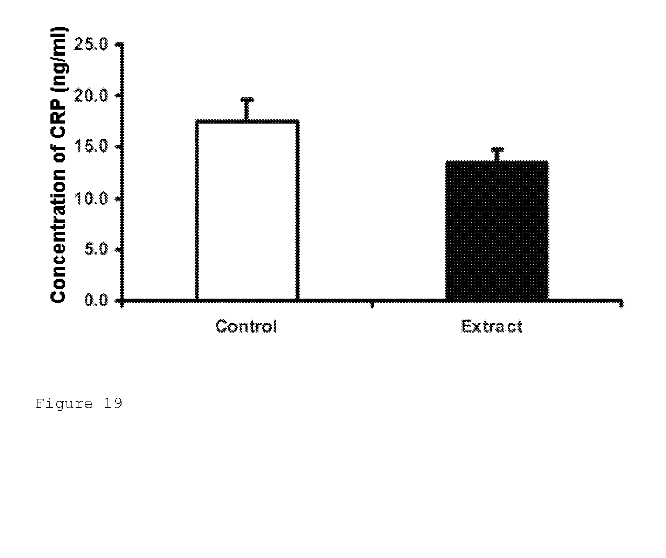

As already explained previously, the serum levels of C-reactive protein in the experimental animals were measured by means of the ELISA technique. According to Figure 19, the group which received extract had on average lower serum levels, which suggests that the use of extract is not accompanied by relevant systemic inflammatory effects.

## Claims

1. An extract of an annelid of the *Lumbricidae* family.

2. An extract according to claim 1, wherein the annelid belongs to the genus *Allolobophora.*

3. An extract according to claim 2, wherein the annelid belongs to the species *Allolobophora caliginosa.*

4. A method for obtaining an extract of an annelid of the *Lumbricidae* family comprising the steps of
(i) maintaining at least one annelid of the *Lumbricidae* family at a temperature of 20 to 25°C and
(ii) collecting the liquid released by the annelid

5. A method according to claim 4, wherein the liquid is collected between minutes 2 to 15 counted from the time at which heating at 20-25°C started.

6. A method according to claim 5, wherein the annelid belongs to the genus *Allolobophora.*

7. A method according to claim 6, wherein the annelid belongs to the species *Allolobophora caliginosa.*

8. An extract obtained by means of a method according to any of claims 4 to 7.

9. A pharmaceutical composition comprising an extract of annelid according to any of claims 1 to 3 or 8 and a pharmaceutically acceptable excipient.

10. An extract according to any of claims 1 to 3 or 8 or a pharmaceutical composition according to claim 9 for its use in medicine.

11. An extract according to any of claims 1 to 3 or 8 or a pharmaceutical composition according to claim 9 to promote the healing of a wound.

12. An extract according to claim 11, wherein the wound is selected from the group consisting of an aseptic wound, a contused wound, an incised wound, a lacerated wound, a non-penetrating wound, an open wound, a penetrating wound, a perforating wound, a punctured wound, a septic wound, a subcutaneous wound, an ischemic ulcer, a pressure sore, a fistula, a bite wound, a thermal burn and a donor site wound.

13. A cosmetic method for regenerating the skin comprising administering to an individual an extract according to any of claims 1 to 3 or 8.

14. A material for treating a wound, wherein said material comprises an extract according to any of claims 1 to 3 or 8.

15. A material according to claim 15, wherein the material is a bandage.
